# EUROPEAN PATENT APPLICATION

(11) **EP 4 674 465 A1**
(43) Date of publication of application: **07.01.2026**
(21) Application number: 24810435.8
(22) Date of filing: 22.05.2024
(51) Int. Cl.: A61M 60/13, A61M 60/178

(54) **CATHETER PUMP AND MOLD**

(30) Priority: 24.05.2023 CN 202321266340 U
(71) Applicant: Magassist Co., Ltd., Suzhou, Jiangsu 215163 (CN)
(72) Inventor: YEN, Ifan, Suzhou, Jiangsu 215163 (CN)
(74) Representative: Winter, Brandl - Partnerschaft mbB
(86) International application number: PCT/CN2024/094790
(87) International publication number: WO 2024/240196

(57) **Abstract**

A catheter pump (1000) and mold, the catheter pump (1000) includes a catheter (201) and a pump head (205), the pump head (205) is delivered to a desired location in heart via the catheter (201) to pump blood; the pump head (205) includes a pump housing (2051) and an impeller (2052) housed within the pump housing (2051), the pump housing (2051) has a blood inlet port (2051a) and a blood outlet port (2051b). The pump housing (2051) includes a stent (20511) which is operable to switch between a radially collapsed state and a radially expanded state; in the radially expanded state, the stent (20511) includes a main body portion (11), an inlet portion (12) located at an axial distal end of the main body portion (11), and an outlet portion (13) located at an axial proximal end of the main body portion (11), the inlet portion (12) and the outlet portion (13) are both connected with the main body portion (11) and support the main body portion (11); a proximal end of the outlet portion (13) is connected with a proximal connecting portion (14), a distal end of the inlet portion (12) is connected with a distal connecting portion (15). At least one of the proximal connecting portion (14) and the distal connecting portion (15) includes a preformed member (16); in a natural state where the stent (20511) is not assembled to the catheter pump (1000), a radial distance from an inner end of the preformed member (16) to a central axis (17) of the stent (20511) is less than a radial distance from an outer end of the preformed member (16) to the central axis (17) of the stent (20511).

## Description

The present invention claims priority to Patent Application No. 202321266340X, entitled "Catheter Pump and Mold for Shape-setting a Stent of the Catheter Pump", filed with the China National Intellectual Property Administration on May 24, 2023.

### Technical Field

The present invention relates to the technical field of medical devices, and, in particular, relates to a catheter pump and mold.

### Background

Catheter pumps are classified into non-collapsible and collapsible types. A collapsible catheter pump creates a smaller entry wound during insertion, offering the advantage of faster, more convenient use.

A collapsible catheter pump has a collapsible pump head that consists of a pump housing defined by a covering membrane and a stent, with an impeller located inside the stent. The stent is the key component enabling collapsibility. Typically, the stent includes a pump section which is in middle and is roughly cylindrical, an inlet section and an outlet section which are located at the two axial ends of the pump section and are roughly conical. Struts in the inlet section and the outlet section connect to and support the pump section, so that its shape is maintained. Most of the impeller lies within the pump section, and a specific clearance must be kept between the pump section and the impeller to avoid rubbing or collision during rotation of the impeller. For optimal hydraulic performance, this clearance needs to remain stable.

In practice, however, the clearance between the pump section and the impeller cannot stay at the desired stable value because the stent is subjected to lateral forces. For example, these lateral forces can be radially inward when the pump head swings inside the ventricle and strikes the ventricular wall, or radially outward when they are generated by the back-pressure of the blood as the impeller rotates to pump blood.

Testing shows that the stent's lateral stiffness is weak. Either or both of the above situations can easily produce radial deformation of the stent, destabilizing the clearance.

### Summary

In view of the deficiencies of the art known to inventors, the object of the present invention is to provide a catheter pump and a mold that can increase the lateral stiffness of the stent when expanded, enhance the ability of the stent to resist lateral forces, and thereby keep the pump clearance stable.

A catheter pump, including a catheter and a pump head, the pump head is delivered to a desired location in the heart via the catheter to pump blood; the pump head includes a pump housing and an impeller housed within the pump housing, the pump housing has a blood inlet port and a blood outlet port; the impeller is driven to rotate to draw blood into the pump housing through the blood inlet port and then expel blood through the blood outlet port.

The pump housing includes a stent, the stent is operable to switch between a radially collapsed state and a radially expanded state; in the radially expanded state, the stent includes a main body portion, an inlet portion located at an axial distal end of the main body portion, and an outlet portion located at an axial proximal end of the main body portion, the inlet portion and the outlet portion are both connected with the main body portion and support the main body portion; a proximal end of the outlet portion is connected with a proximal connecting portion, a distal end of the inlet portion is connected with a distal connecting portion. At least one of the proximal connecting portion and the distal connecting portion includes a preformed member; in a natural state where the stent is not assembled to the catheter pump, a radial distance from an inner end of the preformed member to a central axis of the stent is less than a radial distance from an outer end of the preformed member to the central axis of the stent.

Preferably, after the stent is assembled to the catheter pump, a radial distance from any point on the preformed member to the central axis of the stent is uniform along an axial direction of the stent.

Preferably, the proximal connecting portion includes a plurality of proximal connecting legs spaced apart along a circumferential direction of the stent; at least one of the plurality of proximal connecting legs is formed as the preformed member; the remaining proximal connecting legs other than the preformed member, a radial distance from the proximal connecting leg to the central axis of the stent is uniform along an axial direction of the stent in the natural state.

Preferably, in the plurality of proximal connecting legs, the number of the preformed member is half of the total number of the plurality of proximal connecting legs, one proximal connecting leg extending along the axial direction of the stent is disposed between two adjacent preformed member. Or, all the proximal connecting legs are the preformed members.

Preferably, the distal connecting portion includes a plurality of distal connecting legs spaced apart along the circumferential direction of the stent; at least one of the plurality of distal connecting legs is formed as the preformed member; the remaining distal connecting legs other than the preformed member, the radial distance from any point on the distal connecting leg to the central axis of the stent is uniform along the axial direction of the stent in the natural state.

Preferably, in the plurality of distal connecting legs, the number of the preformed member is half of the total number of the plurality of distal connecting legs, one distal connecting leg extending along the axial direction of the stent is disposed between two adjacent preformed member. Or, all the distal connecting legs are the preformed members.

Preferably, after the stent is assembled to the catheter pump, a tubular restraining member is sleeved outside the preformed member, so that the preformed member maintains a restrained state in which the radial distance from the preformed member to the central axis of the stent is uniform; when the tubular restraining member is removed, the preformed member returns to the natural state.

Preferably, in the natural state, a radial distance from the preformed member to the central axis of the stent gradually increases in a direction from the inner end of the preformed member to the outer end thereof.

Preferably, the gradual increase is linear increase or curved increase.

Preferably, in the natural state, all the preformed member are of equal length.

Preferably, the angle between each preformed member and the central axis of the stent is the same.

A mold is used for shape-setting the stent of the catheter pump described in any of the above embodiments; the mold includes an inner shaping mold, the inner shaping mold includes a middle section, two taper-shaped sections respectively located at two axial ends of the middle section, and two flared sections; the two flared sections are disposed in one-to-one correspondence with the two taper-shaped sections, each of the two flared sections is disposed axially outside of its corresponding taper-shaped section; wherein, the middle section is used to shape-form the main body portion of the stent, the two taper-shaped sections are respectively used to shape-form the inlet portion and the outlet portion of the stent, the flared section is used to shape-form the preformed member.

Preferably, the mold further includes an outer shaping mold detachably covering the outside of the inner shaping mold, the outer shaping mold has an inner mold cavity, the shape of the inner mold cavity is adapted to the shape of the external contour of the inner shaping mold.

The catheter pump provided by this embodiment is characterized in that at least one of the proximal connecting portion and the distal connection portion of the stent includes a preformed member. In the natural state of the stent before it is assembled to the catheter pump, the radial distance from the inner end of the preformed member to the central axis of the stent is less than the radial distance from the outer end of the preformed member to the central axis of the stent. Namely, in the state where the stent is unconstrained by any object and free of any external force, the end of the preformed member away from main body portion flares outwardly. After the stent is assembled to the catheter pump, the preformed member is constrained so that its radial distance to the central axis becomes uniform everywhere. By modifying the structure of the stent, the outward-flared preformed member is collapsed inward while the stent is in its radially expanded state, this induces an inward torsion and creates pre-stress within the stent, thereby increasing its lateral stiffness upon expansion, enhancing its ability to resist lateral forces, and consequently maintaining a stable pump clearance.

### Brief Description of the Drawings

The accompanying drawings, which form a part of the present invention, are intended to provide a further understanding of the present invention. The illustrative embodiments of the present invention and the descriptions thereof are used to explain the present invention, and do not constitute an improper limitation on the present invention. In the drawings:
Fig. 1 shows a schematic structural diagram of a stent in the natural state according to an embodiment of the present invention;

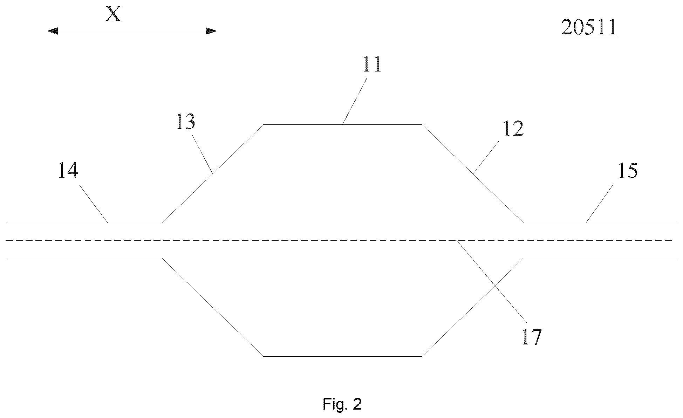

Fig. 2 shows a schematic structural diagram of a stent in the radially expanded state according to an embodiment of the present invention;
Fig. 3 shows a schematic structural diagram of a catheter pump according to an embodiment of the present invention;
Fig. 4 shows a schematic structural diagram of a catheter pump according to another embodiment of the present invention;
Fig. 5 shows a partial sectional view of Fig. 4;
Fig. 6 to Fig. 9 show schematic structural diagrams of a mold shaping a stent according to an embodiment of the present invention;
Fig. 10 shows a partial enlarged view of the upper part of Fig. 5;
Fig. 11 shows a partial enlarged view of the middle part of Fig. 5;
Fig. 12 shows a partial enlarged view of the lower part of Fig. 5.

The above-mentioned drawings include the following reference numerals:
1000, catheter pump;
100, power assembly; 101, housing;
200, working assembly; 201, catheter;
202, driving shaft; 2021, flexible shaft; 2022, rigid shaft;
204, driving catheter handle;
205, pump head; 2051, pump housing; 2051a, blood inlet port; 2051b, blood outlet port;
20511, stent; 20512, covering membrane; 2052, impeller; 20521, hub; 20522, blade;
206, proximal bearing chamber; 207, distal bearing chamber; 208, proximal bearing;
209, distal bearing; 210, atraumatic support member; 211, stop; 212, retainer;
11, main body portion; 12, inlet portion; 13, outlet portion; 14, proximal connecting portion;
15, distal connecting portion; 16, preformed member; 17, central axis; 18, cylindrical structure;
3, inner shaping mold; 31, middle section; 32, taper-shaped section; 33, flared section;
4, outer shaping mold; 41, inner mold cavity;
63, proximal ferrule; 73, distal ferrule.

### Detailed Description of the Embodiments

The terms "proximal", "distal", and "front", "rear" used in this invention are defined with respect to the clinician who manipulates the catheter pump 1000 of the present embodiment. The terms "proximal" and "rear" refer to the portion relatively closer to the clinician, whereas "distal" and "front" refer to the portion relatively farther from the clinician. For example, the extracorporeal segment is at the proximal end or rear end, while the segment intervened inside the body is at the distal end or front end.

Refer to Figs .1 and 2, the stent 20511 of the present invention is operable to switch between a radially collapsed state and a radially expanded state. In the radially expanded state, the stent 20511 includes a main body portion 11, an inlet portion 12 and an outlet portion 13, the main body portion 11 is generally cylindrical; along the axial direction X, the two ends of the main body portion 11 are its distal end and proximal end, respectively. The inlet portion 12 and the outlet portion 13 are located at the distal end and proximal end of the main body portion 11, respectively. The inlet portion 12 and the outlet portion 13 both are generally conical in shape. The inlet portion 12 and the outlet portion 13 are both connected with the main body portion 11 and support the main body portion 11. Along the axial direction X, the two ends of the outlet portion 13 are its proximal end and distal end, respectively, and the two ends of the inlet portion 12 are its proximal end and distal end, respectively. The proximal end of the outlet portion 13 is connected with a proximal connecting portion 14, the distal end of the inlet portion 12 is connected with a distal connecting portion 15.

It should be noted that the radial direction of the stent 20511 is perpendicular to the axial direction X; along the axial direction X, the two ends of the stent 20511 are its proximal end and distal end, respectively.

The proximal connecting portion 14 and the distal connecting portion 15 are used to connect the stent 20511 to other components at its proximal end and distal end, respectively. For example, the proximal connecting portion 14 can connect the stent 20511 with the proximal bearing chamber 206 or the catheter 201, the distal connecting portion 15 can connect the stent 20511 with the distal bearing chamber 207 or the atraumatic support member 210.

In the present embodiment, at least one of the proximal connecting portion 14 and the distal connecting portion 15 includes a preformed member 16. In a natural state, in the radial direction of the stent 20511, the distance from the inner end of the preformed member 16 to the central axis 17 of the stent 20511 is less than the distance from the outer end of the preformed member 16 to the central axis 17 of the stent 20511; that is, the radial distance from the inner end of the preformed member 16 to the central axis of the stent 20511 is less than the radial distance from the outer end of the preformed member 16 to the central axis of the stent 20511.

Specifically, along the axial direction X and in the direction from the inner end to the outer end of the preformed member 16, the preformed member 16 gradually moves away from the central axis 17 of the stent 20511.

Along the axial direction X, the two ends of the preformed member 16 are its inner end and outer end, respectively. The inner end of the preformed member 16 is the end portion connected with the inlet portion 12 or the outlet portion 13, that is, the inner end of the preformed member 16 is used to connect with the proximal end of the outlet portion 13 or the distal end of the inlet portion 12. The outer end of the preformed member 16 is the end portion that faces away from the inlet portion 12 or the outlet portion 13.Concretely, for the preformed member 16 located at the proximal end of the outlet portion 13, the inner end of the preformed member 16 is its distal end, and the outer end of the preformed member 16 is its proximal end; for the preformed member 16 located at the distal end of the inlet portion 12, the inner end of the preformed member 16 is its proximal end, and the outer end of the preformed member 16 is its distal end.

The natural state is a state in which the stent 20511 is not assembled to the catheter pump 1000, also can be the state of the stent 20511 before it is assembled to the catheter pump 1000. Specifically, in the natural state, the proximal connecting portion 14 of the stent 20511 is not yet assembled with the catheter 201 or the proximal bearing chamber 206, the distal connecting portion 15 of the stent 20511 is not yet assembled with the distal bearing chamber 207 or the atraumatic support member 210. In the natural state, the stent 20511 is free from any constraint and thus subjected to no external forces, the outer end of the preformed member 16 (the end far away from the main body portion 11) flares outward. After the stent 20511 is assembled to the catheter pump 1000, a radial distance from the preformed member 16 to the central axis 17 of the stent 20511 is uniform along the axial direction of the stent 20511. By modifying the structure of the stent 20511, the outward-flared preformed member 16 is collapsed inward while the stent 20511 is in its radially expanded state, this induces an inward torsion and creates pre-stress within the stent 20511, thereby increasing its lateral stiffness upon expansion, enhancing its ability to resist lateral forces, and consequently maintaining a stable pump clearance. Wherein, the "central axis 17" is the axis that extends along axial direction X and passes through the center of the stent 20511.

It should be noted that, the axial direction of the stent 20511 is parallel to, or identical with, the axial direction X.

In the art known to inventors, the two ends of the stent (corresponding to the positions of the proximal connecting portion 14 and the distal connecting portion 15 of the present invention) maintain a cylindrical profile both before and after installation. After the external constraint is removed, the middle cylindrical section self-expands from its collapsed configuration to expansion configuration through the material's shape-memory effect. This self-expansion is essentially a stress-release process, full expansion configuration of the stent is reached when the stored stress has been almost completely dissipated. Consequently, once expanded, the stent is in a zero-stress state or near-zero-stress state and inherently possesses low stiffness against lateral deformation. In contrast, the stent 20511 of the present embodiment retains internal pre-stress after expansion, and this pre-stress significantly increases lateral stiffness of the stent 20511.

In this embodiment, the term "stiffness" specifically refers to the ability of stent 20511 to resist deformation under radially outward forces while in the radially expanded state (particularly during operation). The greater the stiffness of stent 20511, the better its ability to resist deformation under radially outward forces; or, equivalently, the smaller the degree of inward radial deformation under the same radially outward force. Conversely, the lower the stiffness of stent 20511, the poorer its ability to resist deformation under radially outward forces, or, equivalently, the greater the degree of inward radial deformation under the same radially outward force.

Specifically, as described below, when the pump head 205 of the catheter pump 1000 employing the stent 20511 of this embodiment is introduced into the ventricle and the impeller 2052 rotates to pump blood, the pump head 205 may swing within the ventricle due to certain factors such as patient movement or cardiac motion, resulting in a possibility of lateral impact against the ventricular wall. If the stent 20511 lacks sufficient stiffness, such lateral impact may cause the stent 20511 to deform radially inward, which may further lead to the rotating impeller 2052 scraping against the stent 20511. This unintended situation is undesirable, as it may cause the impeller 2052 to become entangled with the stent 20511, potentially forcing the impeller 2052 to stop and resulting in pump failure.

At least one of the proximal connecting portion 14 and the distal connecting portion 15 includes a preformed member 16; in the radially expanded state, the flared preformed member 16 is expanded inward, causing the stent 20511 to twist inward and thereby develop a pre-stress. This pre-stress increases the lateral stiffness of the stent 20511 when expanded, enhances the ability to resist lateral forces of the stent 20511, and thus keeps the pump clearance stable. As a result, the probability that the pump head 205 will undergo radial inward collapse upon lateral impact is greatly reduced, preventing the impeller 2052 from contacting the inner wall of the stent 20511 and avoiding the pump-blood failure caused by the impeller 2052 being forced to stop.

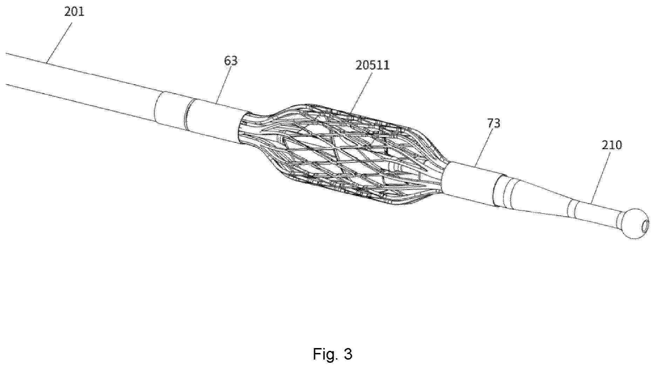

The proximal connecting portion 14 includes a plurality of proximal connecting legs (not shown) spaced apart along a circumferential direction of the stent 20511; the proximal connecting leg is used to connect with the catheter 201 or the proximal bearing chamber 206, thereby securing the stent 20511 to the catheter 201. Specifically, as shown in Fig. 3, the outer wall of the proximal bearing chamber 206 is configured with a plurality of proximal leg slots (not shown) that receive the plurality of proximal connecting legs in one-to-one correspondence. A proximal ferrule 63 is fitted over the proximal connecting portion 14; the proximal ferrule 63 is used for fixing the proximal connecting leg along the radially direction of the stent 20511, so that each of the plurality of proximal connecting legs is kept seated in its corresponding proximal leg slot at all times.

It should be noted that the circumferential direction of the stent 20511 is disposed around its central axis 17.

At least one of the plurality of proximal connecting legs is formed as the preformed member 16; the remaining proximal connecting legs other than the preformed member 16, the radial distance from the proximal connecting leg to the central axis 17 of the stent 20511 is uniform along the axial direction of the stent 20511 in the natural state. That is, in the natural state, only the preformed member 16 of all the proximal connecting legs flares outward, while all other proximal connecting legs remain parallel to the central axis 17 of the stent 20511.

The present embodiment employs a structure of proximally distributed connecting legs, it allows the main body portion 11 of the stent 20511 to remain small-sized in the radially collapsed state, yet ensures a large expanded diameter and high supporting stiffness when the main body portion 11 of the stent 20511 is in the radially expanded state. Moreover, compared with a stent that is carved from a large-diameter pre-formed tube and has a connecting collar at its proximal end, the stent 20511 of the present embodiment generates no scrap material, offers lower cost, and involves simpler fabrication.

A plurality of proximal connecting legs are spaced evenly along the circumferential direction of the stent 20511 and are of equal length, guaranteeing stable connection.

In some embodiments, in the plurality of proximal connecting legs, the number of the preformed member 16 is half of the total number of the plurality of proximal connecting legs, and one proximal connecting leg extending along the axial direction X or the axial direction of the stent 20511 is disposed between two adjacent preformed member 16. That is, in the proximal connecting portion 14, legs that flare outward in the natural state and legs that do not flare are disposed alternately, so that after expansion the stent 20511 develops pre-stress uniformly along its circumferential direction.

In some embodiments, all the proximal connecting legs are the preformed members 16. That is, all the proximal connecting legs flare outward in the natural state, the proximal connecting portion 14 is generally shaped like an outward-flared trumpet. With this arrangement, the proximal connecting portion 14 can maximize the pre-stress generated in the expanded stent 20511, thereby maximizing the lateral stiffness of stent 20511 upon expansion, and enhancing the ability of stent 20511 to resist lateral forces.

The distal connecting portion 15 includes a plurality of distal connecting legs (not shown) spaced apart along the circumferential direction of the stent 20511; the proximal connecting leg is used to connect with the atraumatic support member 210 or the distal bearing chamber 207. As described above and shown in Fig. 3, the outer wall of the atraumatic support member 210 or the distal bearing chamber 207 is configured with a plurality of distal leg slots (not shown), the plurality of distal connecting legs are inserted one-to-one into the plurality of distal leg slots. A distal ferrule 73 is fitted over the atraumatic support member 210 or the distal bearing chamber 207, the distal ferrule 73 is used for fixing the distal connecting leg along the radially direction of the stent 20511, so that each of the plurality of distal connecting legs is kept seated in its corresponding distal leg slot at all times.

At least one of the plurality of distal connecting legs is formed as the preformed member 16; the remaining distal connecting legs other than the preformed member 16, the radial distance from the distal connecting leg to the central axis 17 of the stent 20511 is uniform along the axial direction of the stent 20511 in the natural state. That is, in the natural state, only the preformed member 16 of all the distal connecting legs flares outward, while all other distal connecting legs remain parallel to the central axis 17 of the stent 20511. A plurality of distal connecting legs are spaced evenly along the circumferential direction of the stent 20511 and are of equal length, guaranteeing stable connection.

In some embodiments, in the plurality of distal connecting legs, the number of the preformed member 16 is half of the total number of the plurality of distal connecting legs, one distal connecting leg extending along the axial direction X or the axial direction of the stent 20511 is disposed between two adjacent preformed member 16. That is, in the distal connecting portion 15, legs that flare outward in the natural state and legs that do not flare are disposed alternately, so that after expansion the stent 20511 develops pre-stress uniformly along its circumferential direction.

In some embodiments, all the distal connecting legs are the preformed members 16. That is, all the distal connecting legs flare outward in the natural state, the distal connecting portion 15 is generally shaped like an outward-flared trumpet. With this arrangement, the distal connecting portion 15 can maximize the pre-stress generated in the expanded stent 20511, thereby maximizing the lateral stiffness of stent 20511 upon expansion, and enhancing the ability of stent 20511 to resist lateral forces.

In some embodiments, all the proximal connecting legs are the preformed members 16, and simultaneously all the distal connecting legs are the preformed members 16, thus, in the radially expanded state, both the proximal and the distal preformed members 16 are collapsed inward, causing the proximal end and distal end of the stent 20511 to twist inward and generate pre-stress, thereby maximizing the pre-stress generated in the expanded stent 20511, better increasing the lateral stiffness of stent 20511 upon expansion , and enhancing the ability of stent 20511 to resist lateral forces.

After the stent 20511 is assembled to the catheter pump, a tubular restraining member is sleeved outside the preformed member 16, so that the preformed member 16 maintains a restrained state in which the radial distance from the preformed member 16 to the central axis 17 of the stent 20511 is uniform. Wherein, the tubular restraining member corresponds to the afore-mentioned proximal ferrule 63 and distal ferrule 73, it serves to maintain the installed state of the preformed member 16 and prevent the preformed member 16 from springing back under its own elasticity. when the tubular restraining member is removed, the preformed member 16 returns to the natural state, namely the outward-flared state; thereby ensuring that the preformed member 16 continuously exerts an outward pre-stress on the remaining portions of the stent 20511 throughout the entire working period.

In the natural state, along the axial direction X, a radial distance from the preformed member 16 to the central axis 17 of the stent 20511 gradually increases in a direction from the inner end of the preformed member 16 to the outer end thereof. Preferably, the gradual increase is linear increase or curved increase. When the gradual increase is linear increase, the preformed member 16 in the natural state extends essentially in a straight line, the straight linear preformed member 16 is easy to manufacture, while raising the lateral stiffness of the stent 20511 upon expansion, simplifies the fabrication process. When the gradual increase is curved increase (for example, is quadratic curved increase), the preformed member 16 in the natural state is curved.

Preferably, in the natural state, all the preformed member 16 are of equal length. Furthermore, the angle between each preformed member 16 and the central axis 17 of the stent 20511 is the same, so that the stent 20511 develops a uniformly distributed pre-stress in its circumferential direction after expansion.

Unlike the structures known to the inventors in the art, in which both ends have cylindrical profiles; the present stent 20511, before installation, has ends that flare outward in a trumpet shape. After installation, these trumpet-shaped ends are compressed inward, causing the two ends of the stent 20511 to drive the end structures of the inlet portion 12, the outlet portion 13 and the middle main body portion 11 to twist inward. As a result, the installed stent 20511 develops pre-stress in the inlet portion 12, the outlet portion 13 and the main body portion 11.

The catheter pump 1000 of the present invention is used to partially take over the blood-pumping function of the heart. In scenarios suitable for left-ventricular assistance, the catheter pump 1000 draws blood from the left ventricle and delivers it into the aorta, thereby supporting systemic circulation, reducing the workload of the subject's heart, or providing additional continuous pumping power when the heart's own pumping capacity is insufficient. Naturally, the catheter pump 1000 can also be introduced, via an interventional procedure, to any other desired target location within the subject, such as the right ventricle, a blood vessel, or the interior of another organ.

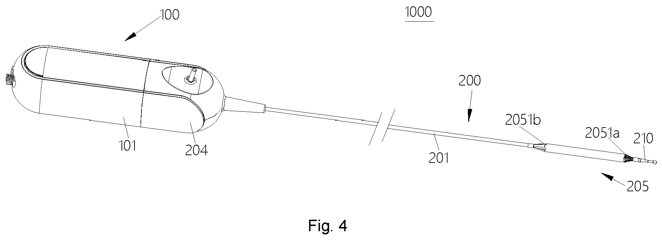

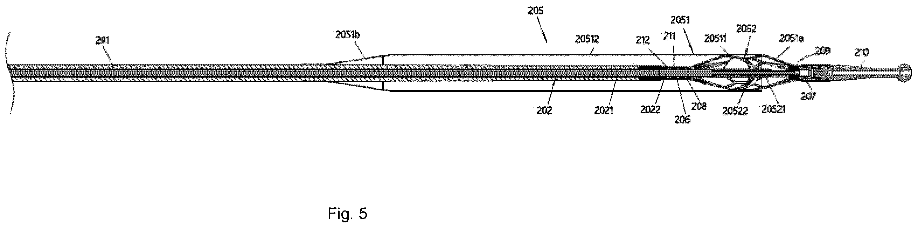

As shown in Figs. 3 and 4, the catheter pump 1000 includes a power assembly 100 and a working assembly 200. The power assembly 100 includes a housing 101, a motor (not shown) housed inside the housing 101, and a driving member (not shown) driven by the motor. With further reference to Figs. 5 and 10 to12, the working assembly 200 includes a catheter 201, a drive shaft 202 extending through the catheter 201, a driven member connected to the proximal end of the drive shaft 202, and a driving catheter handle 204 and a pump head 205 connected to the proximal and distal ends of the catheter 201, respectively. The pump head 205 can be delivered to a desired location in the heart (e.g., the left ventricle for blood pumping) via the catheter 201, the pump head 205 includes a pump housing 2051 and an impeller 2052 housed within the pump housing 2051, the pump housing 2051 has a blood inlet port 2051a and a blood outlet port 2051b. The blood inlet port 2051a is located at the distal end of the pump housing 2051, the blood outlet port 2051b is located at the proximal end of the pump housing 2051. The motor is disposed at the proximal end of the catheter 201 and drives the impeller 2052 to rotate for blood pumping via the drive shaft 202. The impeller 2052 is connected to the distal end of the drive shaft 202. When the impeller 2052 rotates, blood is drawn into the pump housing 2051 through the blood inlet port 2051a and then expelled from the pump housing 2051 through the blood outlet port 2051b.

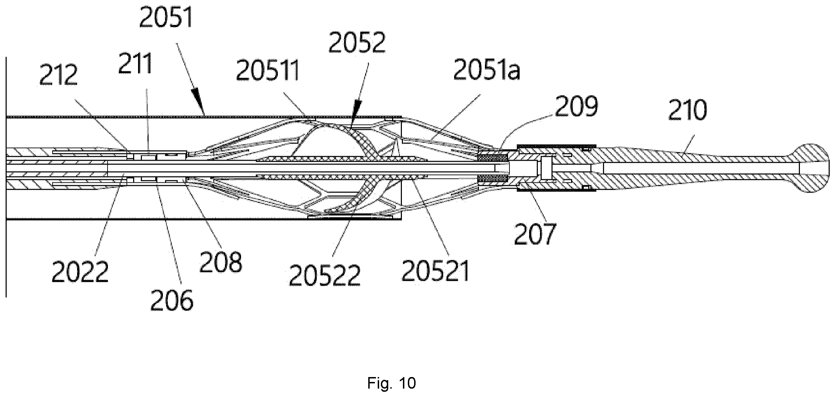

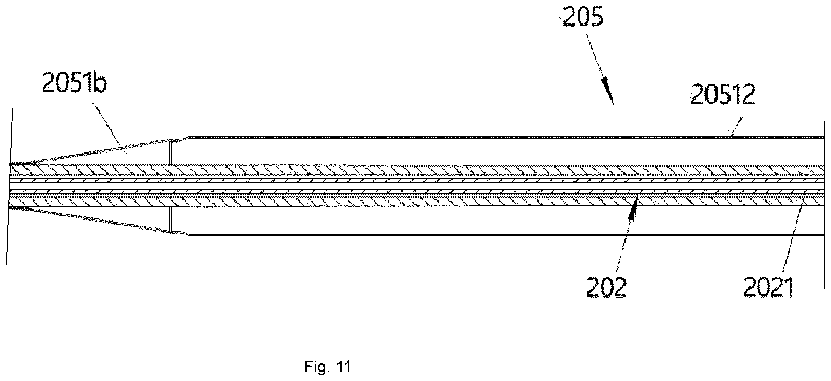

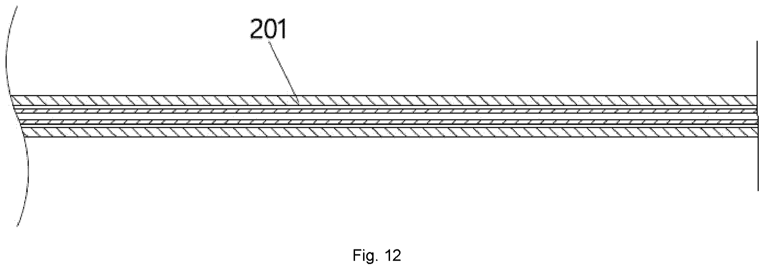

The upper part of Fig. 5 shown in Fig. 10 includes an atraumatic support member 210, a distal bearing 209, a distal bearing chamber 207, a blood inlet port 2051a, a hub 20521, blades 20522, a stent 20511,a proximal bearing 208, a proximal bearing chamber 206, a stop 211, a retainer 212, and a rigid shaft 2022. The middle part of Fig. 5 shown in Fig. 11 includes a covering membrane 20512, a flexible shaft 2021, a drive shaft 202, and a blood outlet port 2051b. The lower part of Fig. 5 shown in Fig. 12 includes a catheter 201.

It should be noted that, the two ends of the drive shaft 202 are its distal end and proximal end, respectively; the pump housing 2051 has two opposite ends, which are its distal end and proximal end, respectively; the two ends of the catheter 201 are its distal end and proximal end, respectively.

The pump housing 2051 is connected to the distal end of the catheter 201, and the impeller 2052 is connected to the distal end of the drive shaft 202. The pump housing 2051 includes a covering membrane 20512 that defines a blood-flow lumen and a collapsible stent 20511 that supports and expands the covering membrane 20512; the proximal end of the stent 20511 is connected with the distal end of the catheter 201. This stent 20511 is the stent 20511 described in any of the preceding embodiments, and the proximal connecting portion 14 of the stent 20511 is connected with the distal end of the catheter 201.

The covering membrane 20512 is elastic and overlies the outer surface of part of the stent 20511. The impeller 2052 is housed inside the stent 20511 and within the covering membrane 20512; the stent 20511 supports the distal end of the covering membrane 20512, with one segment of the stent 20511 lying outside the distal end of the covering membrane 20512 and another segment lying inside the covering membrane 20512. Therein, most of the impeller 2052 is positioned within the main body portion 11 of the stent 20511, the two ends of the impeller 2052 (chiefly the hub 20521) extend into the inlet portion 12 and the outlet portion 13, respectively.

It should be noted that, the covering membrane 20512 has two opposite ends, one of which is its distal end; the direction in which these two opposite ends of the covering membrane 20512 are arranged is the same as the direction in which the two ends of the stent 20511 are arranged.

The covering membrane 20512 can cover the middle and rear portions of the stent 20511; the meshes of the stent 20511 that are not covered by the covering membrane 20512 at the front end form the blood inlet port 2051a. The rear end of the covering membrane 20512 envelops the exterior of the distal end of the catheter 201, and the blood outlet port 2051b is an opening formed at the rear end of the covering membrane 20512.

It should be noted that, the proximal end of the stent 20511 is its rear end, the distal end of the stent 20511 is its front end; the proximal end of the covering membrane 20512 is its rear end.

The covering membrane 20512 has a cylindrical segment that serves as its main body and a tapered segment situated at the proximal end of the cylindrical segment. One end of the cylindrical segment is its proximal end, and one end of the tapered segment is also its proximal end. The proximal end of the tapered segment is positioned around the exterior of the catheter 201 and is fixed to the outer wall of the catheter 201. The catheter 201 connects to the proximal end of the stent 20511 through the proximal bearing chamber 206 located at its distal end, the proximal bearing chamber 206 houses the proximal bearing 208 that rotatably supports the drive shaft 202.

The impeller 2052 includes a hub 20521 and blades 20522 supported on the outer wall of the hub 20521. The blades 20522 are made of a flexible material, and the stent 20511 is made of a nickel-titanium memory alloy; the blades 20522, the stent 20511 and the covering membrane 20512 together form a collapsible pump head 205.

A distal bearing chamber 207 is provided at the distal end of the stent 20511; the distal bearing chamber 207 houses a distal bearing 209 that rotatably supports the distal end of the drive shaft 202. The drive shaft 202 includes a flexible shaft 2021 and a rigid shaft 2022. The flexible shaft 2021 runs through the catheter 201 and is bendable; the rigid shaft 2022 passes through the hollow lumen of the hub 20521 and is connected to the distal end of the flexible shaft 2021, one end of the flexible shaft 2021 is its distal end. The hub 20521 of the impeller 2052 is sleeved onto the rigid shaft 2022, the proximal and distal ends of the rigid shaft 2022 are respectively inserted into the proximal bearing 208 and the distal bearing 209; the two ends of the rigid shaft 2022 are its proximal end and distal end, respectively. Thus, with both ends of the rigid shaft 2022 supported by two bearings and with its high rigidity, the rigid shaft 2022 furnishes stiff support for the impeller 2052 inside the pump housing 2051, keeping the impeller 2052 well retained and stably positioned within the pump housing 2051.

The proximal bearing 208 has a proximal side and a distal side along its axis. A stop 211 is disposed on the rigid shaft 2022 at the proximal side of the proximal bearing 208; the stop 211 limits movement of the rigid shaft 2022 and the impeller 2052 toward the distal side of the proximal bearing 208, preventing the impeller 2052 from moving toward the distal side of the proximal bearing 208 under the reverse force of blood during rotation for pumping. The direction from the proximal side to the distal side of the stop 211 is the same as the direction from the proximal side to the distal side of the proximal bearing 208. A retainer 212 is also disposed on the rigid shaft 2022 at the proximal side of the stop 211; the retainer 212 limits movement of the rigid shaft 2022 and the stop 211 toward the proximal side of the stop 211, preventing the stop 211 from abrading the distal end of the catheter 201 and releasing particulate debris.

One axial end of the distal bearing chamber 207 is its distal end. An atraumatic support member 210 made of flexible material is disposed at the distal end of the distal bearing chamber 207, the atraumatic support member 210 props gently against the ventricular wall in a non-traumatic or atraumatic manner, keeping the blood inlet port 2051a of the pump head 205 spaced from the ventricular wall, preventing the blood inlet port 2051a of the pump head 205 from being pressed against the ventricular wall by the reverse force of blood during operation of the pump head 205, thereby ensuring an effective suction area.

The driving catheter handle 204 and the power assembly100 are detachably connected; the connection can be achieved by a locking nut or by the snap-fit coupling disclosed in US9421311B2. A driven member and a driving member are coupled without contact, so that the rotary power of the motor is transmitted to the drive shaft 202, thereby driving the impeller 2052 to rotate and pump blood. As described above, the driven member and the driving member can adopt the magnetic coupling disclosed in CN103120810B or CN101820933B, or the Eddy Current Coupling disclosed in CN216061675U or CN114452527A; the present embodiment imposes no limitation thereon.

The above-described catheter pump 1000 employs an external motor. On the basis of the foregoing, the catheter pump 1000 can alternatively adopt a built-in-motor configuration. In that case, the motor is connected to the distal end of the catheter 201, the elongated flexible drive shaft 202 is no longer threaded through the catheter 201, and the motor drives the impeller 2052 by means of a short rigid shaft, magnetic coupling, or the like.

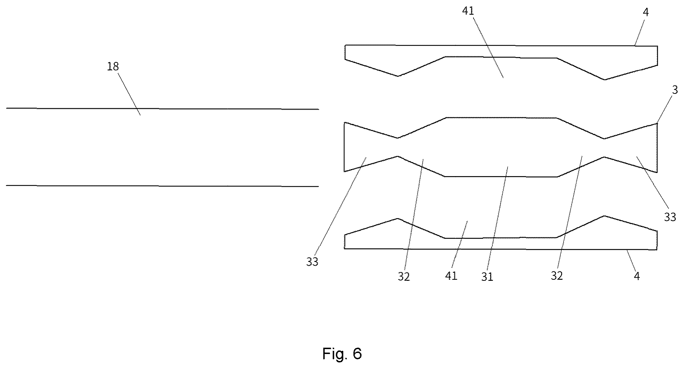

Refer to Figs. 6 to 9. The present invention further provides a mold, the mold is used for shape-setting the stent 20511 of the catheter pump 1000 described in any of the above embodiments. The mold includes an inner shaping mold 3. The inner shaping mold 3 includes a substantially cylindrical middle section 31, two taper-shaped sections 32 respectively located at two axial ends of the middle section 31, and the flared section 33 disposed axially outside of the taper-shaped section 32. Namely, the inner shaping mold 3 includes two flared sections 33, the two flared sections 33 are disposed in one-to-one correspondence with the two taper-shaped sections 32, each of the two flared sections 33 is disposed axially outside of its corresponding taper-shaped section 32. The middle section 31 is used to shape-form the main body portion 11, the two taper-shaped sections 32 are respectively used to shape-form the inlet portion 12 and the outlet portion 13, the flared section 33 is substantially trumpet-shaped and is used to shape-form the preformed member 16.

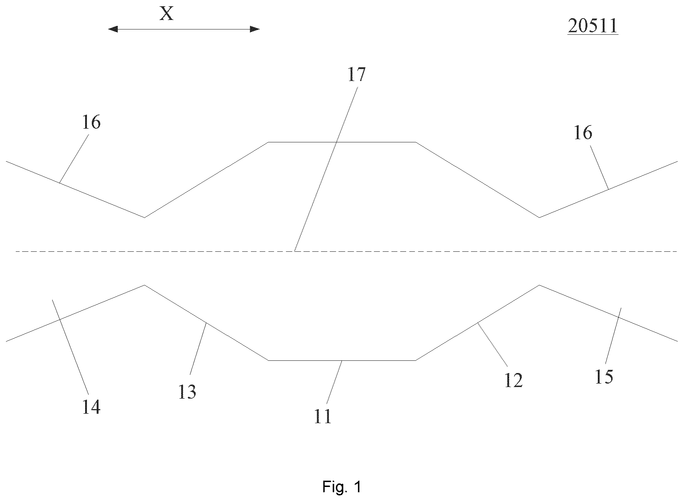

The mold further includes an outer shaping mold 4 detachably covering the outside of the inner shaping mold 3, the outer shaping mold 4 has an inner mold cavity, the shape of the inner mold cavity is adapted to the shape of the external contour of the inner shaping mold 3. The shape of the external contour of the inner shaping mold 3 and the shape of the inner mold cavity of the outer shaping mold 4 can be referred to the shape of the stent 20511 shown in Fig. 1. The outer shaping mold 4 is a Half mold to enable detachability from the inner shaping mold 3.

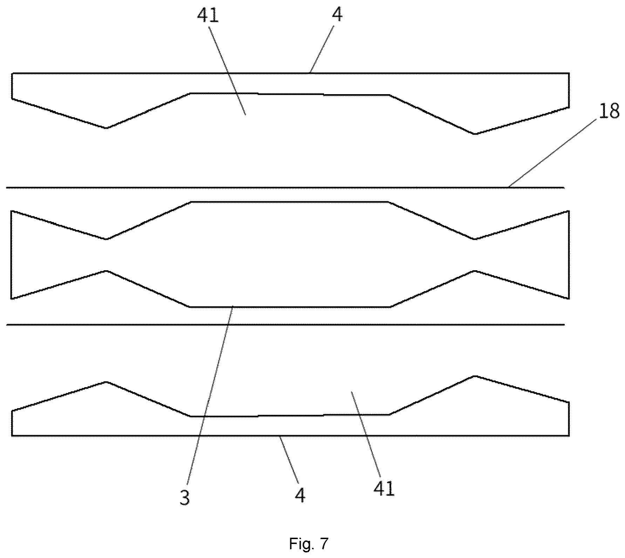

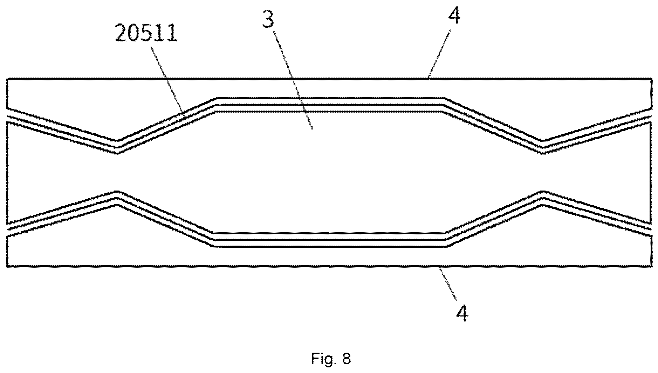

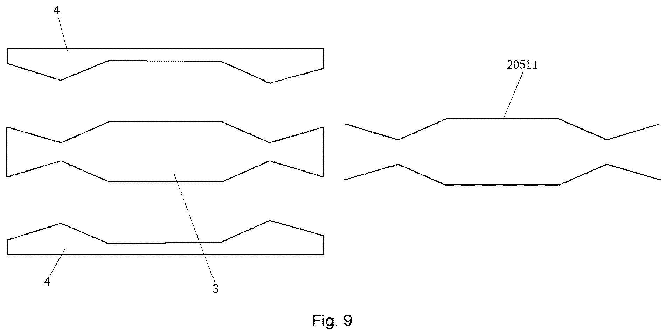

In a specific application scenario, the stent 20511 is first fabricated from a single pre-formed tube. This single pre-formed tube is laser-cut in its entirety to produce the hollow mesh-patterned cylindrical structure 18 shown in Fig. 6 (i.e., the stent before shaping; at this stage no distinction is yet made among the main body portion 11, the proximal connecting portion 14, the distal connecting portion 15, etc.). The outer diameter of the cylindrical structure 18 is uniform along its entire axial length. Subsequently, this hollow cylindrical structure 18 is subjected to a shaping process to obtain the final stent 20511. Specifically, as shown in Fig. 7, the pre-shaped stent (cylindrical structure 18) is slipped over the inner shaping mold 3; then, as shown in Fig. 8, the outer shaping mold 4 is fitted over the inner shaping mold 3. Thereafter, the pre-shaped stent undergoes a heat-treatment process, it is heated to the phase-transformation temperature of the stent material (e.g., nitinol), held at that temperature for a period of time, cooled, and then demolded, yielding the final stent 20511 shown in Fig. 9.

The above embodiments merely illustrate several implementations of the present invention. Although the description is relatively specific and detailed, it should not be construed as limiting the scope of the invention. It should be pointed out that, without departing from the concept of the present invention, those skilled in the art can make various variations and improvements, all of which fall within the protection scope of the present invention. Therefore, the protection scope of the present invention shall be subject to the appended claims.

## Claims

1. A catheter pump, wherein the catheter pump comprises a catheter and a pump head, the pump head is delivered to a desired location in heart via the catheter to pump blood; the pump head comprises a pump housing and an impeller housed within the pump housing, the pump housing has a blood inlet port and a blood outlet port; the impeller is driven to rotate to draw blood into the pump housing through the blood inlet port and then expel blood through the blood outlet port;
the pump housing comprises a stent, the stent is operable to switch between a radially collapsed state and a radially expanded state; in the radially expanded state, the stent comprises a main body portion, an inlet portion located at an axial distal end of the main body portion, and an outlet portion located at an axial proximal end of the main body portion, the inlet portion and the outlet portion are both connected with the main body portion and support the main body portion; a proximal end of the outlet portion is connected with a proximal connecting portion, a distal end of the inlet portion is connected with a distal connecting portion;
wherein, at least one of the proximal connecting portion and the distal connecting portion comprises a preformed member; in a natural state, a radial distance from an inner end of the preformed member to a central axis of the stent is less than a radial distance from an outer end of the preformed member to the central axis of the stent; the natural state is a state in which the stent is not assembled to the catheter pump.

2. The catheter pump according to claim 1, wherein after the stent is assembled to the catheter pump, a radial distance from the preformed member to the central axis of the stent is uniform along an axial direction of the stent.

3. The catheter pump according to claim 1, wherein the proximal connecting portion comprises a plurality of proximal connecting legs spaced apart along a circumferential direction of the stent; at least one of the plurality of proximal connecting legs is formed as the preformed member; remaining proximal connecting legs other than the preformed member, a radial distance from a proximal connecting leg to the central axis of the stent is uniform along an axial direction of the stent in the natural state.

4. The catheter pump according to claim 3, wherein,
in the plurality of proximal connecting legs, a number of the preformed member is half of a total number of the plurality of proximal connecting legs, one proximal connecting leg extending along the axial direction of the stent is disposed between two adjacent preformed member; or all the plurality of proximal connecting legs are the preformed members.

5. The catheter pump according to claim 1, wherein the distal connecting portion comprises a plurality of distal connecting legs spaced apart along a circumferential direction of the stent; at least one of the plurality of distal connecting legs is formed as the preformed member; remaining distal connecting legs other than the preformed member, a radial distance from a distal connecting leg to the central axis of the stent is uniform along an axial direction of the stent in the natural state.

6. The catheter pump according to claim 5, wherein,
in the plurality of distal connecting legs, a number of the preformed member is half of a total number of the plurality of distal connecting legs, one distal connecting leg extending along the axial direction of the stent is disposed between two adjacent preformed member; or
all the plurality of distal connecting legs are the preformed members.

7. The catheter pump according to claim 2 or 5, wherein after the stent is assembled to the catheter pump, a tubular restraining member is sleeved outside the preformed member, so that the preformed member maintains a restrained state in which the radial distance from the preformed member to the central axis of the stent is uniform; when the tubular restraining member is removed, the preformed member returns to the natural state.

8. The catheter pump according to claim 1, wherein in the natural state, a radial distance from the preformed member to the central axis of the stent gradually increases in a direction from the inner end of the preformed member to the outer end of the preformed member.

9. A mold, wherein, the mold is used for shape-setting the stent of the catheter pump according to any one of claims 1 to 8; wherein the mold comprises an inner shaping mold, the inner shaping mold comprises a middle section, two taper-shaped sections respectively located at two axial ends of the middle section, and two flared sections; the two flared sections are disposed in one-to-one correspondence with the two taper-shaped sections, each of the two flared sections is disposed axially outside of its corresponding taper-shaped section; wherein, the middle section is used to shape-form the main body portion of the stent, the two taper-shaped sections are respectively used to shape-form the inlet portion and the outlet portion of the stent, a flared section is used to shape-form the preformed member.

10. The mold according to claim 9, wherein the mold further comprises an outer shaping mold detachably covering an outside of the inner shaping mold, the outer shaping mold has an inner mold cavity, a shape of the inner mold cavity is adapted to a shape of an external contour of the inner shaping mold.
